# EUROPÄISCHE PATENTSCHRIFT

(11) **EP 1 296 968 B1**
(45) Veröffentlichungstag und Bekanntmachung des Hinweises auf die Patenterteilung: **03.11.2004**
(21) Anmeldenummer: 01969340.7
(22) Anmeldetag: 05.07.2001
(51) Int. Cl.: C07D 301/12

(54) **VERFAHREN ZUR HERSTELLUNG VON PROPYLENOXID**
METHOD FOR THE PRODUCTION OF PROPYLENE OXIDE
PROCEDE DE PRODUCTION D'OXYDE DE PROPYLENE

(30) Priorität: 06.07.2000 DE 10032884
(43) Veröffentlichungstag der Anmeldung: 02.04.2003
(73) Patentinhaber: BASF AKTIENGESELLSCHAFT, 67056 Ludwigshafen (DE)
(72) Erfinder: TELES, Joaquim, Henrique, 67166 Otterstadt (DE); REHFINGER, Alwin, 67112 Mutterstadt (DE); BASSLER, Peter, 68519 Viernheim (DE); BERG, Anne, 2170 Merksem (BE); RIEBER, Norbert, 68259 Mannheim (DE); RUDOLF, Peter, 68526 Ladenburg (DE)
(74) Vertreter: Isenbruck, Günter, Dr.
(86) Internationale Anmeldenummer: PCT/EP2001/007716
(87) Internationale Veröffentlichungsnummer: WO 2002/002544

(56) Entgegenhaltungen:
- EP-A- 0 719 768
- US-A- 5 107 002

## Beschreibung

Die vorliegende Erfindung betrifft ein Verfahren zur Herstellung von Propylenoxid aus Propen und Wasserstoffperoxid in Gegenwart von Methanol. Im Rahmen des erfindungsgemäßen Verfahrens wird nach der Umsetzung des Propens mit Wasserstoffperoxid ein Gemisch, das Methanol, Wasser und nicht-umgesetztes Wasserstoffperoxid enthält, vom Reaktionsaustrag abgetrennt und dieses Gemisch einem Trennverfahren unterworfen, bei dem ein weiteres Gemisch resultiert, das Methanol und Methylformiat enthält.

Verfahren zur Herstellung von Propylenoxid aus Propen sind aus dem Stand der Technik bekannt. Bei diesen Verfahren tritt in der Regel das Problem auf, daß bei der Reaktion eine gewisse Menge an Wasserstoffperoxid nicht umgesetzt wird und bei der anschließenden Abtrennung von Propylenoxid aus dem Reaktionsaustrag anfällt.

Um diesem Problem abzuhelfen, wurde unter anderem vorgeschlagen, Wasserstoffperoxid in einer Zwischenabtrennung aus dem Reaktionsaustrag einer ersten Reaktionsstufe abzutrennen und in einer zweiten Reaktionsstufe erneut mit Alken umsetzen. Solche Verfahren sind beispielsweise in der PCT/EP99/05740 und der DE-A 100 15 246.5 beschrieben. Hierbei ist es zwar möglich, in der zweiten Reaktionsstufe einen nahezu hundertprozentigen Wasserstoffperoxidumsatz zu erreichen. Wegen der zweiten Reaktionsstufe ist jedoch ein erhöhter apparativer Aufwand erforderlich.

Eine Aufgabe der vorliegenden Erfindung war es, ein Verfahren bereitzustellen, in dem das Problem des anfallenden nicht-umgesetzten Wasserstoffperoxides kostengünstig und effizient gelöst wird.

Demgemäß betrifft die vorliegende Erfindung ein Verfahren zur Herstellung von Propylenoxid, in dem
(i) Propen mit Wasserstoffperoxid in Gegenwart von Methanol zu Propylenoxid unter Erhalt eines Gemisches (Gi), umfassend Propylenoxid, Methanol, Wasser und nicht-umgesetztes Wasserstoffperoxid, umgesetzt wird,
(ii) aus dem Gemisch (Gi) ein Gemisch (Gii), umfassend Methanol, Wasser und Wasserstoffperoxid, unter Erhalt eines Gemisches (Ga), umfassend Propylenoxid, abgetrennt wird und
(iii) aus dem Gemisch (Gii) Wasser unter Erhalt eines Gemisches (Giii), umfassend Methanol und Methylformiat, abgetrennt wird.

Besonders bevorzugt wird Methanol als Lösungsmittel eingesetzt. Hierbei ist es auch möglich, zusätzlich zu Methanol ein oder mehrere weitere Lösungsmittel einzusetzen. Als solche weiteren Lösungsmittel können prinzipiell alle für die jeweilige Umsetzung geeigneten Lösungsmittel eingesetzt werden. Unter anderem bevorzugt sind beispielsweise
- Wasser,
- Alkohole, bevorzugt niedere Alkohole, weiter bevorzugt Alkohole mit weniger als 6 Kohlenstoffatomen wie beispielsweise Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen.

Die Umsetzung des Propens mit Wasserstoffperoxid findet im Rahmen des erfindungsgemäßen Verfahrens bevorzugt in Gegenwart eines Katalysators statt. Als Katalysatoren zur Umsetzung des Propylens zu Propylenoxid sind prinzipiell alle, bevorzugt alle heterogenen Katalysatoren denkbar, die für die jeweilige Umsetzung geeignet sind.

Bevorzugt werden dabei Katalysatoren verwendet, die ein poröses oxidisches Material wie z.B. einen Zeolith umfassen. Vorzugsweise werden Katalysatoren eingesetzt, die als poröses oxidisches Material ein Titan-, Vanadium-, Chrom-, Nioboder Zirkonium-haltigen Zeolith umfassen.

Im besonderen existieren Zeolithe, die kein Aluminium enthalten und bei denen im Silikatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben beispielsweise in der EP-A 0 311 983 oder der EP-A 0 405 978.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Vanadium-, Chrom-, Niob-, Zirkoniumhaltige Zeolithe mit Pentasil-Zeolith-Struktur geeignet, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WEI-, WEN-, YUG-, ZON-Struktur sowie zu Mischstrukturen aus zwei oder mehr der vorgenannten Strukturen. Denkbar sind für den Einsatz im erfindungsgemäßen Verfahren weiterhin titanhaltige Zeolithe mit der Struktur des UTD-1, CIT-1 oder CIT-5. Als weitere titanhaltige Zeolithe sind solche mit der Struktur des ZSM-48 oder ZSM-12 zu nennen.

Als besonders bevorzugt sind für das erfindungsgemäße Verfahren Ti-Zeolithe mit MFI-, MEL- oder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu beta-Zeolith isomorphen Gerüststruktur zu nennen.

Insbesondere bevorzugt wird im erfindungsgemäßen Verfahren ein heterogener Katalysator, der das titanhaltige Silikalit TS-1 umfaßt, verwendet.

Demgemäß betrifft die vorliegende Erfindung auch ein Verfahren,wie oben beschrieben, das dadurch gekennzeichnet ist, daß zur Herstellung des Propylenoxides ein Zeolithkatalysator, bevorzugt ein Titansilikalit-Katalysator und insbesondere ein Titansilikalit-Katalysator der Struktur TS-1 eingesetzt wird.

Die Abtrennung von Wasser aus dem Gemisch (Gii) wird im erfindungsgemäßen Verfahren bevorzugt destillativ durchgerührt, wobei eine oder auch mehrere Destillationskolonnen verwendet werden können. Bevorzugt werden eine oder zwei Destillationskolonnen eingesetzt. Im Falle, daß keine Wärmerückgewinnung nötig ist, wird bevorzugt eine Destillationskolonne eingesetzt. Zwei oder auch mehr Destillationskolonnen werden bevorzugt dann eingesetzt, wenn eine besonders gute Wärmeintegration im Verfahren gewährleistet werden soll.

Hinsichtlich der physikalischen Parameter wie Temperatur oder Druck bestehen bei der destillativen Abtrennung von Wasser aus dem Gemisch (Gii) keine besonderen Einschränkungen, solange gewährleistet ist, daß bei der Destillation Wasserstoffperoxid abgebaut wird und ein Gemisch (Giii), umfassend Methylformiat und Methanol, erhalten wird.

Wird im erfindungsgemäßen Verfahren zur Abtrennung des Wassers aus dem Gemisch (Gii) nur eine Kolonne eingesetzt, so weist diese bevorzugt mindestens 5, bevorzugt mindestens 20 und weiter bevorzugt mindestens 30 theoretische Böden auf. Bevorzugt wird die Destillation bei Drücken im Bereich von 0,5 bis 40 bar, bevorzugt von 1,0 bis 20 bar und besonders bevorzugt von 2 bis 15 bar durchgeführt.

Werden im erfindungsgemäßen Verfahren zur Abtrennung des Wassers aus dem Gemisch (Gii) zwei Kolonnen eingesetzt, dann werden die Drücke so gewählt, daß mit der Kondensationswärme am Kopf der Kolonnen andere Prozeßströme aufgeheizt werden können. Dies wird beispielsweise dadurch erreicht, daß der Kondensator mindestens einer Kolonne mit beispielsweise Wasser gekühlt wird und das aus der Kühlung resultierende Warmwasser oder der aus der Kühlung resultierende Dampf zur Beheizung eines oder mehrerer Schritte des erfindungsgemäßen Verfahrens oder auch eines oder mehrerer anderer Verfahren eingesetzt wird.

Bevorzugt wird die erste Destillationskolonne bei Drücken im Bereich von 0,5 bis 40 bar und bevorzugt von 1 bis 20 bar betrieben. In einer möglichen Ausführungsform wird die ersten Kolonne bei einem höheren Druckniveau betrieben als die zweite Kolonne. In diesem Fall wird der Sumpf der zweiten Kolonne mit dem Brüden der ersten Kolonnen beheizt. In einer bevorzugten Ausführungsform wird die erste Kolonne bei einem niedrigeren Druckniveau betrieben als die zweite Kolonne. In diesem Fall wird der Sumpf der ersten Kolonne mit dem Brüden der zweiten Kolonne beheizt.

In einer besonders bevorzugten Ausführungsform werden die erste Kolonne bei Drücken im Bereich von 4 bis 9 bar und weiter bevorzugt im Bereich von 6 bis 8 bar und die zweite im Bereich von 11 bis 16 bar und weiter bevorzugt im Bereich von 12 bis 14 bar betrieben. Im Kopf der ersten Kolonne werden im allgemeinen 20 bis 80 %, bevorzugt 30 bis 70 % und besonders bevorzugt 40 bis 60 % des im Gemisch (Gii) enthaltenen Methanols zusammen mit Methylformiat über Kopf abgetrennt. Das Gemisch, das über Sumpf aus der ersten Kolonne erhalten wird, wird als Feed für die zweite Kolonne verwendet. Das Kopfprodukt der zweiten Kolonne umfaßt das restliche Methanol und Methylformiat und das Sumpfprodukt umfaßt Wasser. Das Kopfprodukt der ersten Kolonnen und das Kopfprodukt der zweiten Kolonne werden zum Gemisch (Giii) vereinigt.

Sowohl im Fall der Wasserabtrennung in zwei Kolonnen als auch im Fall der Wasserabtrennung in einer Kolonne werden die Trennbedingungen besonders bevorzugt so gewählt, dass der Wassergehalt im Gemisch (Giii) im allgemeinen weniger als 3 Gew.-%, bevorzugt weniger als 1 Gew.-% und besonders bevorzugt weniger als 0,3 Gew.-% beträgt. Weiter bevorzugt werden die Trennbedingungen derart gewählt, daß der Methanolgehalt im Sumpfabzug weniger als 5 Gew.-%, bevorzugt weniger als 1 Gew.-% und besonders bevorzugt weniger als 0,2 Gew.-% beträgt.

Der Sumpfabzug kann daneben als weitere Komponenten unter anderem beispielsweise Methoxypropanole, Propylenglykol, Ameisensäure, Dipropylenglykolemonomethylether, Formaldehyd enthalten.

Demgemäß betrifft die vorliegende Erfindung auch ein Verfahren, wie oben beschrieben, in dem das Wasser gemäß (iii) destillativ abgetrennt wird, dadurch gekennzeichnet, daß
(w) aus dem Gemisch (Gii) in einer ersten Destillationskolonne über Kopf ein Gemisch (Gw) abgetrennt wird, das hauptsächlich Methanol und Methylformiat umfaßt,
(x) das aus der ersten Destillationskolonne über Sumpf erhaltene Gemisch als Feed einer zweiten Destillationskolonne zugeführt wird,
(y) aus der zweiten Destillationskolonne über Kopf ein Gemisch (Gy) erhalten wird, das hauptsächlich Methanol und Methylformiat umfaßt, und
(z) die Gemische (Gw) und (Gy) unter Erhalt des Gemisches (Giii) vereinigt werden.

Das Gemisch (Giii), das nach der Abtrennung von Wasser erhalten wird, wird in einer bevorzugten Ausführungsform des erfindungsgemäßen Verfahrens einer weiteren Aufarbeitungsstufe zugeführt. Dabei wird aus dem Gemisch (Giii) Methanol von Methylformiat abgetrennt.

In einer weiter bevorzugten Ausführungsform des erfindungsgemäßen Verfahrens wird das so gewonnene Methanol in (i) rückgeführt.

Daher betrifft die vorliegende Erfindung auch ein Verfahren, wie oben beschrieben, das dadurch gekennzeichnet ist, daß aus dem Gemisch (Giii) Methanol von Methylformiat abgetrennt wird und das abgetrennte Methanol in (i) rückgeführt wird.

Die Abtrennung des Methanols aus dem Gemisch (Giii), umfassend Methylformiat und Methanol, kann hierbei prinzipiell gemäß allen denkbaren Verfahren bewerkstelligt werden, solange gewährleistet ist, daß die Reinheit des abgetrennten Methanols den gestellten Anforderungen genügt.

Unter anderem sind hierbei chemische Methoden zu nennen. Beispielsweise ist es etwa möglich, das Gemisch, umfassend Methanol und Methylformiat, mit einem geeigneten basischen Ionentauscher in Kontakt zu bringen, wodurch Methanol entsteht und das Formiat am Ionentauscher verbleibt. Dieses Verfahren ist unter anderem in der US-A 5,107,002 beschrieben.

Weiter kann das Gemisch, umfassend Methanol und Methylformiat, mit einer Base behandelt werden, wobei das Methylformiat hydrolisiert wird. Dabei sind sämtliche Basen verwendbar, durch die die Hydrolyse des Methylformiats erreicht werden kann. Bevorzugt werden starke Basen eingesetzt. Als besonders bevorzugte Basen sind im Rahmen der vorliegenden Erfindung Salze von Säuren zu nennen, die schwächere Säuren als Ameisensäure sind. Unter anderem bevorzugt sind hierbei etwa Alkali- und Erdalkalihydroxide oder Alkalisalze von Alkoholen oder Phenolen zu nennen. Selbstverständlich können auch Mischungen aus zwei oder mehr dieser Basen eingesetzt werden.

Weiter bevorzugt sind zur Abtrennung von Methanol aus dem Gemisch, umfassend Methanol und Methylformiat, physikalische Methoden wie beispielsweise Destillationsverfahren.

Unter diesen sind beispielsweise Extraktivdestillationsverfahren möglich, wie sie aus dem Stand der Technik bekannt und beispielsweise in der oben genannten USA 5,107,002 aufgeführt sind.

Bevorzugt werden jedoch Destillationsverfahren eingesetzt, die apparativ weniger aufwendig zu realisieren sind als die genannten Extraktionsdestillationsverfahren.

Bevorzugt wird ein Destillationsverfahren, in der eine oder mehrere Kolonnen, weiter bevorzugt eine Kolonne, eingesetzt wird. Wird eine Kolonne eingesetzt, so weist diese mindestens 5, bevorzugt mindestens 10 und insbesondere mindestens 20 theoretische Böden auf.

Die Drücke, bei denen bevorzugt gearbeitet wird, liegen im allgemeinen im Bereich von 0,2 bis 50 bar, bevorzugt im Bereich von 1,5 bis 30 bar und insbesondere im Bereich von 2,0 bis 20 bar.

Die Kopftemperaturen und Sumpftemperaturen werden vom gewählten Druck eindeutig bestimmt. In einer besonders bevorzugten Ausführungsform wird diese Kolonne, die ungefähr 20 theoretische Trennstufen aufweist, bei Drücken im Bereich von 2,0 bis 20 bar betrieben. Als Kopfprodukt erhält man ein Gemisch, umfassend Methylformiat und einen geringen Anteil des im Feed enthaltenen Methanols. Bevorzugt weist dieses Gemisch einen Methanolanteil von weniger als 80 Gew.-%, bevorzugt von weniger als 50 Gew.-% und besonders bevorzugt von weniger als 20 Gew.-% auf.

Weiter ist es denkbar, daß das Gemisch (Giii), umfassend Methanol und Methylformiat, neben Methylformiat zusätzlich weitere Komponenten aufweist. Der Begriff "Komponenten" bezeichnet hierbei sowohl reine Verbindungen als auch Azeotrope, die einen Siedepunkt aufweisen, der niedriger ist als der Siedepunkt von Methanol. Als solche Komponenten sind unter anderem beispielhaft Acetaldehyd, 1,1-Dimethoxyethan, Propionaldehyd, 1,1-Dimethoxypropan, Aceton oder 2,4-Dimethyl-1,3-dioxolan zu nennen. Diese können ebenfalls im Rahmen der Aufarbeitung aus dem Gemisch abgetrennt werden.

So ist es möglich, diese Nebenprodukte vor der.Trennung des Methanols von Methylformiat durch eine oder mehrere geeignete physikalische oder chemische Methoden aus dem Gemisch abzutrennen. Ebenso ist es möglich, zuerst Methanol aus dem Gemisch abzutrennen, wobei ein Gemisch resultieren kann, das Methanol und mindestens eine Verunreinigung enthält. In diesem Fall können sich an die Abtrennung von Methanol aus dem Gemisch eine oder mehrere Abtrennstufen anschließen, in denen Methanol von der mindestens einen Verunreinigung abgetrennt wird. Ebenso kann nach der Abtrennung von Methanol aus dem Gemisch ein Gemisch resultieren, das Methylformiat und eine oder, mehrere Verunreinigungen enthält. Auch dieses kann, falls erforderlich, durch eine oder mehrere geeignete physikalische oder chemische Methoden in seine Bestandteile getrennt werden. Die Bestandteile können dann getrennt oder zusammen einem oder mehreren weiteren Verfahren als Edukte zugeführt oder thermisch verwertet werden.

Je nach chemischer Natur der Verunreinigungen ist es auch möglich, Methanol derart aus dem Gemisch abzutrennen, daß sowohl Methylformiat als auch die mindestens eine Verunreinigung in einem einzigen Verfahrensschritt von Methanol abgetrennt werden.

Durch die wie oben beschriebene, erfindungsgemäß bevorzugt eingesetzte Destillation wird dabei eine Methanolfraktion erhalten, die einen Gehalt an Methylformiat von im allgemeinen weniger als 500 ppm, bevorzugt weniger als 100 ppm und insbesondere bevorzugt weniger als 20 ppm aufweist.

In Abhängigkeit von den Anforderungen, die an die Reinheit der Methanolfraktion gestellt werden, können Reste von anderen Komponenten wie beispielsweise Acetaldehyd, 1,1-Dimethoxyethan, Propionaldehyd, 1,1-Dimethoxypropan, Aceton oder 2,4-Dimethyl-1,3-dioxolan, die im Anschluß an die destillative Aufarbeitung in der Methanolfraktion verbleiben, durch eine oder mehrere geeignete Maßnahmen wie beispielsweise eine oder mehrere weitere Destillationen von Methanol abgetrennt werden.

Im allgemeinen ist es völlig ausreichend, wenn die Konzentration jeder einzelne Nebenkomponente im Methanol unter 1 Gew.-% liegt und die Summe aller Nebenkomponenten 5 Gew.-% nicht überschreitet.

Das derart von Methylformiat abgetrennte Methanol kann wiederverwendet werden, wobei es prinzipiell denkbar ist, das Methanol in das Verfahren zur Herstellung des Propylenoxides rückzuführen oder bei Bedarf einem davon unterschiedlichen Verfahren, in dem Methanol als Lösungsmittel oder als Edukt oder in sonstiger Funktion benötigt wird, zuzuführen. Selbstverständlich ist es denkbar, den Methanolstrom, der aus der erfindungsgemäßen Abtrennung resultiert, in zwei oder mehr Ströme aufzuteilen und jeden Strom einem anderen Verfahren zuzuführen.

In einer besonders bevorzugten Ausführungsform des erfindungsgemäßen Verfahrens wird das Methanol, das von Methylformiat und gegebenenfalls von einem oder mehreren Nebenprodukten oder Verunreinigungen getrennt wurde, wie oben beschrieben in das Verfahren zur Herstellung des Propylenoxides rückgeführt. Unter anderem bevorzugt wird das Methanol in einen Puffertank gepumpt und daraus in das Verfahren eingeschleust.

Was die Herstellung des Propylenoxids gemäß (i) anbelangt, so wird diese ganz besonders bevorzugt einstufig durchgeführt. Der Begriff "einstufig", wie er im Rahmen der vorliegenden Anmeldung verwendet wird, bezeichnet hierbei Verfahrensführungen, bei denen keine Abtrennung von Wasserstoffperoxid stattfindet. Unter anderem umfaßt damit ein einstufiges Verfahren im Rahmen der vorliegenden Erfindung neben Verfahren, in denen die Edukte in einem Reaktor miteinander umgesetzt werden und der Reaktionsaustrag weiterverarbeitet wird, unter anderem auch Verfahren, in denen
- Propen in einer Reaktionsstufe mit Wasserstoffperoxid unter Erhalt eines Produktstroms umgesetzt wird,
- der Produktstrom mindestens einer Zwischenbehandlung zugeführt wird, wobei aus der Zwischenbehandlung ein weiterer Produktstrom erhalten wird, und
- der weitere Produktstrom einer weiteren Reaktionsstufe zugeführt wird, in der Wasserstoffperoxid mit Propen umgesetzt wird,
wobei die Zwischenbehandlungen keine Wasserstoffperoxidabtrennungen sind.

Selbstverständlich sind auch noch weitere Reaktionsstufen denkbar, wobei zwischen zwei Reaktionsstufen eine Zwischenbehandlung erfolgen kann, aber nicht muß. Als Zwischenbehandlung ist unter anderem etwa die Zugabe einer Base zu dem Produktstrom denkbar, wobei insbesondere bevorzugt basische Verbindungen eingesetzt werden können, die im Rahmen des erfindungsgemäßen Verfahrens in gewünschter Weise die Umsetzung von Wasserstoffperoxid mit Propen beeinflussen. Werden beispielsweise Zeolithe als heterogene Katalysatoren eingesetzt, so werden bevorzugt basische Verbindungen eingesetzt, die die Acidität dieser Zeolithe erniedrigen. Solche Basen sind etwa in der DE-A 100 15 246.5 beschrieben, die diesbezüglich vollumfänglich durch Bezugnahme in den Kontext der vorliegenden Anmeldung einbezogen wird.

Was beispielsweise die Anordnung und die Art der Reaktoren anbelangt, die in (i) eingesetzt werden, so sind auch hier sämtliche geeigneten Reaktoren denkbar. Insbesondere können beispielsweise in einer oder mehr der vorgenannten Reaktionsstufen zwei oder mehr parallel geschaltete Reaktoren eingesetzt werden. Diesbezüglich sei auf die DE-A 100 15 246.5 verwiesen, die im Hinblick auf die möglichen Reaktoranordnungen in den Kontext der vorliegenden Anmeldung vollumfänglich durch Bezugnahme einbezogen wird.

Daher betrifft die vorliegende Erfindung ein Verfahren, wie oben beschrieben, das dadurch gekennzeichnet ist, daß die Umsetzung gemäß (i) einstufig durchgeführt wird.

Weiter können im Laufe der Herstellung des Propylenoxides aus Propen und Wasserstoffperoxid während des Verfahrens Temperatur und Druck des Reaktionsmediums geändert werden. Ebenso können der pH-Wert und die Temperatur des Reaktionsmediums geändert werden. Weiter ist es möglich, zusätzlich zu pH-Wert und Temperatur des Reaktionsmediums zusätzlich den Druck zu ändern, unter dem die Reaktion stattfindet. Diesbezüglich sei auf die DE-A 199 36 547.4 verwiesen, die diesbezüglich vollumfänglich durch Bezugnahme in den Kontext der vorliegenden Anmeldung einbezogen wird.

Insbesondere bevorzugt wird die Herstellung des Propylenoxides gemäß (i) derart durchgeführt, daß der Umsatz von Wasserstoffperoxid im allgemeinen im Bereich von 85 bis 99,99 %, bevorzugt im Bereich von 90 bis 99,9 % und besonders bevorzugt im Bereich von 95 bis 99,5 % liegt.

Daher betrifft die vorliegende Erfindung auch ein Verfahren, wie oben beschrieben, das dadurch gekennzeichnet ist, daß der Wasserstoffperoxidumsatz in (i) im Bereich von 85 bis 99,99 liegt.

Erfindungsgemäß wird aus dem Gemisch (Gi) ein Gemisch (Gii), umfassend Methanol, Wasser und Wasserstoffperoxid, unter Erhalt eines Gemisches (Ga), umfassend Propylenoxid, abgetrennt. Diese Abtrennung kann generell nach sämtlichen geeigneten Methoden erfolgen. Bevorzugt wird die Abtrennung destillativ durchgeführt.

Bevorzugt wird ein Destillationsverfahren, in dem eine oder mehrere Kolonneneingesetzt werden, weiter bevorzugt eine Kolonne eingesetzt wird. Wird eine Kolonne eingesetzt, so weist diese mindestens 5, bevorzugt mindestens 10 und insbesondere mindestens 15 theoretischen Böden auf. Die Drücke, bei denen bevorzugt gearbeitet wird, liegen im allgemeinen im Bereich von 0,5 bis 10 bar, bevorzugt im Bereich von 0,8 bis 3 bar und insbesondere im Bereich des Umgebungsdruckes.

Die Kopf und Sumpftemperaturen werden vom gewählten Druck eindeutig bestimmt. In einer ganz besonders bevorzugten Ausführungsform wird diese Kolonne, die ca. 15 theoretische Trennstufen aufweist, bei Umgebungsdruck betrieben. Als Kopfprodukt erhält man bei einer Kopftemperatur im Bereich von ungefähr 35 °C ein Gemisch (Ga), umfassend Propylenoxid. Als Sumpfprodukt erhält man bei einer Sumpftemperatur von ungefähr 68 °C ein Gemisch (Gii), umfassend Methanol, Wasser, nicht-umgesetztes Wasserstoffperoxid.

Das Gemisch (Gii) kann hierbei, zusätzlich zu Methanol, Wasser und Wasserstoffperoxid, ein oder mehrere weitere Verbindungen enthalten, die beispielsweise Nebenprodukte der Reaktion gemäß (i) oder Verbindungen, die bei der Abtrennung gemäß (ii) gebildet werden, oder Verbindungen, die als Verunreinigungen der Edukte in (i) eingeführt wurden, oder auch Lösungsmittel, die beispielsweise zusätzlich zum Lösungsmittel Methanol eingesetzt wurden, oder Verbindungen, die zur destillativen Abtrennung gemäß (ii) eingesetzt wurden, oder Verbindungen, die beispielsweise im Rahmen einer wie oben beschriebenen Zwischenbehandlung zugegeben wurden, sein können. Bei der Herstellung von Propylenoxid aus Propen gemäß (i) kann das Gemisch (Gii) unter anderem 1,1-Dimethoxyethan, Aceton, Acetaldehyd, 1,1-Dimethoxypropan, Propionaldehyd, Methylformiat oder/und 2,4-Dimethyl-1,3-dioxolan enthalten.

Auch das Gemisch (Ga) kann zusätzlich zu Propylenoxid ein oder mehrere weitere Verbindungen enthalten, die wiederum beispielsweise Nebenprodukte der Reaktion gemäß (i) oder Verbindungen, die bei der Abtrennung gemäß (ii) gebildet werden, oder Verbindungen, die als Verunreinigungen der Edukte in (i) eingeführt wurden, oder auch Methanol oder Lösungsmittel, die beispielsweise zusätzlich zum Lösungsmittel Methanol eingesetzt wurden, oder Verbindungen, die zur destillativen Abtrennung gemäß (ii) eingesetzt wurden, oder Verbindungen, die beispielsweise im Rahmen einer wie oben beschriebenen Zwischenbehandlung zugegeben wurden, sein können.

Je nach Verfahrensführung ist es im Rahmen des erfindungsgemäßen Verfahrens denkbar, daß das Gemisch (Ga), das aus dem Reaktionsaustrag gemäß (i) erhalten wird, zusätzlich zu Propylenoxid Methanol enthält. Bevorzugt sind Methanolgehalte Im Bereich von 10 bis 90 Gew.-%, besonders bevorzugt im Bereich von 25 bis 75 Gew.-% und ganz besonders bevorzugt im Bereich von 40 bis 60 Gew.-% Methanol.

In einer unter anderem bevorzugten Ausführungsform wird das Methanol, das in (Ga) enthalten ist, aus (Ga) abgetrennt und dem Gemisch (Gii) zugegeben. Der Ausdruck "zum Gemisch (Gii) zugeben" umfaßt im Rahmen der vorliegenden Anmeldung sowohl Ausführungsformen des erfindungsgemäßen Verfahrens, bei denen das aus (Ga) abgetrennte Methanol zuerst (Gii) zugegeben wird und das resultierende Gemisch der Verfahrensstufe (iii) zugeführt wird, als auch Ausführungsformen, bei denen das aus (Ga) abgetrennt Methanol und das Gemisch (Gii) getrennt der Verfahrensstufe (iii) zugeführt werden und erst dort in Kontakt kommen.

Femer kann das Methanol auch dem Gemisch (Giii) zugegeben werden.

Die vorliegende Erfindung betrifft daher auch ein Verfahren, wie oben beschrieben, wobei das Gemisch (Ga) zusätzlich zu Propylenoxid Methanol umfaßt, dadurch gekennzeichnet, daß Methanol aus (Ga) abgetrennt wird und dem Gemisch (Gii) oder dem Gemisch (Giii) oder den Gemischen (Gii) und (Giii) zugegeben wird..

Die Abtrennung des Methanols aus dem Gemisch (Ga), umfassend Methanol und Propylenoxid, erfolgt bevorzugt über ein destillatives Verfahren, wobei die Anzahl der eingesetzten Kolonnen im wesentlichen beliebig ist. Bevorzugt wird eine Kolonne verwendet. Dabei weist diese Kolonne bevorzugt mindestens 20, bevorzugt mindestens 40 und weiter bevorzugt mindestens 60 theoretische Böden auf.

Die Destillation in dieser Kolonne wird bevorzugt bei Drücken im Bereich von 0,3 bis 10 bar, bevorzugt von 0,4 bis 2 bar und besonders bevorzugt von 0,6 bis 1,2 bar durchgeführt.

Die vorliegende Erfindung betrifft daher auch ein Verfahren, bei dem aus (Ga) Methanol abgetrennt wird und wie es oben beschrieben ist, das dadurch gekennzeichnet ist, daß die Abtrennung des Methanols in einer Kolonne mit mindestens 20 theoretischen Böden bei Drücken im Bereich von 0,3 bis 10 bar erfolgt.

Der Reaktionsaustrag aus (i) kann in (i) nicht-umgesetztes Propen enthalten. Dieses wird bevorzugt aus dem Reaktionsaustrag abgetrennt.

In einer bevorzugten Ausführungsform des erfindungsgemäßen Verfahrens erfolgt die Abtrennung des nicht-umgesetzten Propens im Rahmen der Abtrennung des Gemisches (Gii) in der Verfahrensstufe (ii). In einer weiter bevorzugten Ausführungsform wird die destillative Abtrennung gemäß (ii) hierbei derart durchgeführt, daß beispielsweise das Gemisch (Gii) über Sumpf abgetrennt wird, das Gemisch (Ga) über einen Seitenabzug der Destillationskolonne abgetrennt wird und über Kopf das nicht-umgesetzte Propen abgetrennt wird.

In einer ebenfalls bevorzugten Ausführungsform des erfindungsgemäßen Verfahrens wird die Abtrennung gemäß (ii) derart durchgeführt, daß das Gemisch (Ga), das aus (ii) erhalten wird, zusätzlich zu Propylenoxid und gegebenenfalls Methanol in (i) nicht-umgesetztes Propen enthält. Dieses Propen wird im Rahmen des weiteren Verfahrens bevorzugt aus (Ga) abgetrennt.

Daher betrifft die vorliegende Erfindung auch ein Verfahren, wie oben beschrieben, wobei das Gemisch (Ga) neben Propylenoxid und gegebenenfalls Methanol zusätzlich in (i) nicht-umgesetztes Propen umfaßt, das dadurch gekennzeichnet ist, daß das Propen aus dem Gemisch (Ga) abgetrennt wird.

Bevorzugt erfolgt die Abtrennung des nicht-umgesetzten Propens aus (Ga) destillativ. Dabei ist die Anzahl der eingesetzten Kolonnen im wesentlichen beliebig. Bevorzugt wird eine Kolonne verwendet. Diese Kolonne weist im allgemeinen mindestens 5, bevorzugt mindestens 10 und weiter bevorzugt mindestens 15 theoretische Böden auf. Die Destillation in dieser Kolonne wird bevorzugt bei Drücken im Bereich von 0,5 bis 25 bar, bevorzugt von 0,7 bis 5 bar und besonders bevorzugt im Bereich von 0,9 bis 1,5 bar durchgeführt.

Bei der Abtrennung des nicht-umgesetzten Propens tritt unter Umständen das Problem auf, daß beim Abtrennen des Propens als Leichtsiederfraktion, wie oben beschrieben, sich in dieser Leichtsiederfraktion Sauerstoff in einer Konzentration ansammeln kann, die die Leichtsiederfraktion zu einem zündfähigen Gemisch macht. Dadurch kann ein ernstes Sicherheitsrisiko entstehen, wenn Propen wiederum destillativ von der Leichtsiederfraktion abgetrennt wird, was dann bevorzugt der Fall ist, wenn das Propen in (i) rückgeführt werden soll.

Dieses Problem kann beispielsweise dadurch gelöst werden, daß Propen destillativ aus dem Leichtsiedergemisch entfernt wird und im oberen Teil der dafür verwendeten Trenneinrichtung ein inerter Stoff mit einem Siedepunkt, der niedriger als der des Propens ist, bevorzugt Methan, in einer solchen Menge zuzugeben wird, daß der Sauerstoff bis zu einer Konzentration verdünnt ist, bei der das Gemisch nicht mehr zündfähig ist. Dieses Verfahren ist beispielsweise in der EP-B 0 719 768 beschrieben. Bevorzugt wird das Problem jedoch dadurch gelöst, daß ein Verfahren zur Aufarbeitung eines Gemisches, umfassend Propen und Sauerstoff, angewendet wird, in dem Sauerstoff nicht-destillativ aus dem Gemisch unter Erhalt eines weiteren Gemisches entfernt wird und aus dem weiteren Gemisch das Propen destillativ abgetrennt wird. Dieses Verfahren ist in der DE-A 100 01 401.1 beschrieben, die diesbezüglich vollumfänglich durch Bezugnahme in den Kontext der vorliegenden Anmeldung einbezogen wird.

Das abgetrennte Propen wird in einer besonders bevorzugten Ausführungsform des erfindungsgemäßen Verfahrens wieder als Edukt in (i) rückgeführt.

In einer weiter besonders bevorzugten Ausführungsform finden sämtliche der genannten Verfahrensstufen kontinuierlich statt. Selbstverständlich ist es auch möglich, eine oder mehre der Stufen in Batch-Fahrweise zu betreiben.

## Patentansprüche

1. Verfahren zur Herstellung von Propylenoxid, in dem
(i) Propen mit Wasserstoffperoxid in Gegenwart von Methanol zu Propylenoxid unter Erhalt eines Gemisches (Gi), umfassend Propylenoxid, Methanol, Wasser und nicht-umgesetztes Wasserstoffperoxid, umgesetzt wird,
(ii) aus dem Gemisch (Gi) ein Gemisch (Gii), umfassend Methanol, Wasser und Wasserstoffperoxid, unter Erhalt eines Gemisches (Ga), umfassend Propylenoxid, abgetrennt wird und
(iii) aus dem Gemisch (Gii) Wasser unter Erhalt eines Gemisches (Giii), umfassend Methanol und Methylformiat, abgetrennt wird.

2. Verfahren nach Anspruch 1, **dadurch gekennzeichnet, daß** zur Herstellung des Propylenoxides ein Zeolithkatalysator, bevorzugt ein Titansilikalit-Katalysator und insbesondere ein Titansilikalit-Katalysator der Struktur TS-1 eingesetzt wird.

3. Verfahren nach Anspruch 1 oder 2, in dem das Wasser gemäß (iii) destillativ abgetrennt wird, **dadurch gekennzeichnet, daß**
(w) aus dem Gemisch (Gii) in einer ersten Destillationskolonne über Kopf ein Gemisch (Gw) abgetrennt wird, das hauptsächlich Methanol und Methylformiat umfaßt,
(x) das aus der ersten Destillationskolonne über Sumpf erhaltene Gemisch und als Feed einer zweiten Destillationskolonne zugeführt wird,
(y) aus der zweiten Destillationskolonne über Kopf ein Gemisch (Gy) erhalten wird, das hauptsächlich Methanol und Methylformiat umfaßt, und
(z) die Gemische (Gw) und (Gy) unter Erhalt des Gemisches (Giii) vereinigt werden.

4. Verfahren nach einem der Ansprüche 1 bis 3, **dadurch gekennzeichnet, daß** aus dem Gemisch (Giii) Methanol von Methylformiat abgetrennt wird und das abgetrennte Methanol in (i) rückgeführt wird.

5. Verfahren nach einem der Ansprüche 1 bis 4, **dadurch gekennzeichnet, daß** die Umsetzung gemäß (i) einstufig durchgeführt wird.

6. Verfahren nach Anspruch 5, **dadurch gekennzeichnet, daß** der Wasserstoffperoxidumsatz in (i) im Bereich von 85 bis 99,99 % liegt.

7. Verfahren nach einem der Ansprüche 1 bis 6, wobei das Gemisch (Ga) zusätzlich zu Propylenoxid Methanol umfaßt, **dadurch gekennzeichnet, daß** Methanol aus (Ga) abgetrennt wird und dem Gemisch (Gii) oder dem Gemisch (Giii) oder den Gemischen (Gii) und (Giii) zugegeben wird.

8. Verfahren nach Anspruch 7, **dadurch gekennzeichnet, , daß** die Abtrennung des Methanols in einer Kolonne mit mindestens 20 theoretischen Böden bei Drücken im Bereich von 0,3 bis 10 bar erfolgt.

9. Verfahren nach einem der Ansprüche 1 bis 8, wobei das Gemisch (Ga) neben Propylenoxid und gegebenenfalls Methanol zusätzlich in (i) nicht-umgesetztes Propen umfaßt, **dadurch gekennzeichnet, daß** das Propen aus dem Gemisch (Ga) abgetrennt wird.

## Claims

1. A process for the preparation of propylene oxide in which
(i) propene is reacted with hydrogen peroxide in the presence of methanol to give propylene oxide, giving a mixture (Gi) comprising propylene oxide, methanol, water and unreacted hydrogen peroxide,
(ii) a mixture (Gii) comprising methanol, water and hydrogen peroxide is separated off from the mixture (Gi), giving a mixture (Ga) comprising propylene oxide, and
(iii) water is separated off from the mixture (Gii), giving a mixture (Giii) comprising methanol and methyl formate.

2. A process as claimed in claim 1, wherein the propylene oxide is prepared using a zeolite catalyst, preferably a titanium silicalite catalyst and in particular a titanium silicalite catalyst having the TS-1 structure.

3. A process as claimed in claim 1 or 2, in which the water is separated off by distillation in (iii), wherein
(w) a mixture (Gw) which principally comprises methanol and methyl formate is separated off from the mixture (Gii) at the top of a first distillation column,
(x) the mixture obtained at the bottom of the first distillation column is fed as feed to a second distillation column,
(y) a mixture (Gy) which principally comprises methanol and methyl formate is obtained at the top of the second distillation column, and
(z) the mixtures (Gw) and (Gy) are combined to give the mixture (Giii).

4. A process as claimed in one of claims 1 to 3, wherein methanol is separated from methyl formate in the mixture (Giii), and the methanol separated off is recycled into (i).

5. A process as claimed in one of claims 1 to 4, wherein the reaction in (i) is carried out in one step.

6. A process as claimed in claim 5, wherein the hydrogen peroxide conversion in (i) is in the range from 85 to 99.99%.

7. A process as claimed in one of claims 1 to 6, where the mixture (Ga) comprises methanol in addition to propylene oxide, wherein methanol is separated off from (Ga) and added to the mixture (Gii) or the mixture (Giii) or the mixtures (Gii) and (Giii).

8. A process as claimed in claim 7, wherein the removal of the methanol is carried out in a column having at least 20 theoretical plates at pressures in the range from 0.3 to 10 bar.

9. A process as claimed in one of claims 1 to 8, where the mixture (Ga), in addition to propylene oxide and possibly methanol, additionally comprises propene which has not reacted in (i), wherein the propene is separated off from the mixture (Ga).

## Revendications

1. Procédé de préparation d'oxyde de propylène, dans lequel
(i) on fait réagir du propène avec du peroxyde d'hydrogène en présence de méthanol pour donner de l'oxyde de propylène en obtenant un mélange (Gi), comprenant de l'oxyde de propylène, du méthanol, de l'eau et du peroxyde d'hydrogène n'ayant pas réagi,
(ii) on sépare à partir du mélange (Gi) un mélange (Gii) comprenant du méthanol, de l'eau et du peroxyde d'hydrogène, en obtenant un mélange (Ga) comprenant de l'oxyde de propylène, et
(iii) on sépare à partir du mélange (Gii) de l'eau en obtenant un mélange (Giii) comprenant du méthanol et du formiate de méthyle.

2. Procédé selon la revendication 1, **caractérisé en ce qu'**on utilise pour la production de l'oxyde de propylène un catalyseur de zéolithe, de préférence un catalyseur de silicalite de titane et en particulier un catalyseur de silicalite de titane de structure TS-1.

3. Procédé selon la revendication 1 ou 2, dans lequel on sépare par distillation l'eau selon (iii), **caractérisé en ce que**
(w) à partir du mélange (Gii), on sépare dans une première colonne de distillation, par la tête, un mélange (Gw), qui comprend principalement du méthanol et du formiate de méthyle,
(x) on verse le mélange de la première colonne de distillation obtenu par le fond et comme charge à une seconde colonne de distillation,
(y) on obtient à partir de la seconde colonne de distillation, par la tête, un mélange (Gy) qui comprend principalement du méthanol et du formiate de méthyle, et
(z) on réunit les mélanges (Gw) et (Gy) en obtenant le mélange (Giii).

4. Procédé selon l'une des revendications 1 à 3, **caractérisé en ce qu'**à partir du mélange (Giii) on sépare le méthanol du formiate de méthyle et on réintroduit le méthanol séparé dans (i).

5. Procédé selon l'une des revendications 1 à 4, **caractérisé en ce qu'**on conduit la réaction selon (i) en une étape.

6. Procédé selon la revendication 5, **caractérisé en ce que** le taux de transformation du peroxyde d'hydrogène dans (i) se situe dans un intervalle allant de 85 à 99,99%.

7. Procédé selon l'une des revendications 1 à 6, dans lequel le mélange (Ga) comprend, outre l'oxyde de propylène, du méthanol, **caractérisé en ce qu'**on sépare le méthanol de (Ga) et **en ce qu'**on l'ajoute au mélange (Gii) ou au mélange (Giii), ou aux mélanges (Gii) et (Giii).

8. Procédé selon la revendication 7, **caractérisé en ce qu'**on procède à la séparation du méthanol dans une colonne comportant au moins 20 plateaux théoriques à des pressions allant de 0,3 à 10 bars.

9. Procédé selon l'une des revendications 1 à 8, dans lequel le mélange (Ga) contient en outre, à côté de l'oxyde de propylène et le cas échéant du méthanol, du propène n'ayant pas réagi dans (i), **caractérisé en ce qu'**on sépare le propène du mélange (Ga).
